# EUROPEAN PATENT APPLICATION

(11) **EP 1 468 659 A2**
(43) Date of publication of application: **20.10.2004**
(21) Application number: 04290979.6
(22) Date of filing: 13.04.2004
(51) Int. Cl.: A61C 17/022, A61C 3/025

(54) **Dental prophylaxis and air appliance**

(30) Priority: 14.04.2003 US 413173
(71) Applicant: Policicchio, Piero Angelo, Holland, Michigan 49424 (US)
(72) Inventor: Policicchio, Piero Angelo, Holland, Michigan 49424 (US)
(74) Representative: Geismar, Thierry

(57) **Abstract**

A dental prophylaxis and air appliance for in-home use having a pressurized air source (16), a pneumatic or mechanical switching device (20) to turn on and off the air source, a hand piece (12), and a pressurized reservoir for a mixture of dry or wetted abrasive and non-abrasive components. The compressed air is delivered to the hand piece (12) by a line that meters out and discharges the post-mixed water slurry of abrasive or non-abrasive components.

## Description

### FIELD OF THE INVENTION

The present invention generally relates to a dental prophylaxis device for home use and, more particularly, relates to a dental prophylaxis device that is particularly suited for cleaning and providing prophylaxis of teeth, gingiva, and other oral tissue.

### BACKGROUND AND SUMMARY OF THE INVENTION

Over the years, numerous dental devices have been proposed which provide prophylaxis through what is commonly referred to as a "sandblasting" technique. While sometimes intended for home use, these devices are more often only appropriate for office or clinical use because of the additional equipment needed for their operation. This equipment is both complex and expensive.

One of the first devices of the above-mentioned type is disclosed in U.S. Pat. No. 3,972,123. The device of the '123 patent discloses a nozzle which ejects an abrasive laden air stream surrounded by a shroud of warm water. Improving on the '123 patent is U.S. Pat. No. 4,174,571. The '571 patent discloses the use of a water-soluble abrasive in the air stream. Unfortunately, both of these systems are quite elaborate and therefore costly to produce, manufacture, purchase, and maintain, all of which make them not particularly suited for home use.

One general problem with these particular types of devices is that the nozzle of the devices is susceptible to becoming clogged by the abrasive in the air stream and therefore requires frequent maintenance. This problem has led to the development of numerous devices, which utilize some variety of mechanism for agitating of the abrasive material in an attempt to prevent the discharge ports from becoming clogged. For obvious reasons, a device, which is not susceptible to clogging without the added expense of an agitating mechanism, is desirable.

U.S. Pat. No. 4,214,871 discloses the introduction of a soluble abrasive particle into the liquid stream, which is discharged against the teeth and gingiva. In this device, water, at household pressures, is delivered through a nozzle that entrains the abrasive particles into the liquid stream (where they partially dissolve) and ejects them against the teeth and the adjacent oral tissues. Household water pressure, however, has proven to be ineffective at providing sufficient pressures for adequately removing plaque and other calculus. A greater force for propelling the liquid entrained particles is needed.

U.S. Pat. No. 4,776,794 uses a piston applying pressure to push pre-mixed abrasive liquid into an air stream. The device uses a water pipe and a mixing chamber at the tip to incorporate air, water, and premixed slurry. The device was designed for a professional setting and is not readily adaptable for home use since it requires a separate pressurized air source and pre-mixed abrasive slurry. The premixed slurry does not allow additional medicaments to be added by user.

U.S. Pat. Application No. 2003/0013063 discloses a container holding pre-mixed slurry. A bubble foam laden with abrasive particles is forced into a jet stream of air similar to U.S. Pat. No. 5,203,698. A drawback to this appliance is that it uses pre-mixed slurry, which decreases the shelf life of product. In addition, the system is complex and difficult to manufacture.

U.S. Pat No. 5,393,228 employs a system of air, water, and dental cleaner to clean teeth and around gums, whereby air pressurizes a water chamber, which in turn uses water to pressurize a receptacle of dental cleaner forcing the wetted cleaner into a stream of accelerated air flow.

As can be seen from the above discussion, the principal direction of technology in this field has been toward devices, which are better suited for use in the professional dental office where the costs of the equipment necessary for providing compressed air and water are more easily afforded and recovered. Additionally, the prior devices are cumbersome since they require the use of specially prepared abrasives, such as finely milled sodium bicarbonate, an air/powder, or water/powder suspension.

With the limitations of the prior art in mind, it is a principal object of the present invention to provide a dental prophylaxis device which is particularly suited to use in the home of a patient for daily and thorough lavage of the teeth, gingival tissue, and general oral cavity. However, it is also an object of the present invention to provide an air appliance, which is adaptable to alternative uses within the home, including uses unrelated to oral hygiene. The present invention could be used, for example, to operate various other household items, which perform scrubbing, spraying, dispensing, foaming, pumping, or other functions.

The air appliance of the present invention can be used for dermatological applications, recreational applications, toys, propulsion devices, preservation devices, marinating devices, drain opener, fire extinguisher, lather-maker, pressurized cooking devices, nasal and sinus cleansing applications, humidifying device, nebualizing devices, atomizing devices, manicuring applications, massaging device, air delivering tool devices, and painting devices.

The air appliance of the present invention uses a cleaning technique that directs an abrasive-laden fluid stream at the teeth, gingiva, and other oral tissue. The abrasive stream cleans out food particles while removing plaque from the surfaces of the teeth and at the tooth/gingiva interface. Additionally, the devices stimulate circulation in the gingiva and oxygenates various anaerobic bacteria, both of which help to prevent periodontal diseases.

An advantage to the present invention as a home device is the lack of a need for a pressurized water vessel. Another advantage is the user in the home can vary the dilution of the slurry and consequently the level of abrasiveness or efficacy of the cleaning, through the amount of water or other liquid used in wetting the dry components.

Additional benefits and advantages of the present invention will become apparent to those skilled in the art to which this invention relates from the subsequent description of the preferred embodiments and the appended claims, taken in conjunction with the accompanying drawings.

### BRIEF DESCRIPTION OF THE DRAWINGS

The present invention will become more fully understood from the detailed description and the accompanying drawings, wherein:

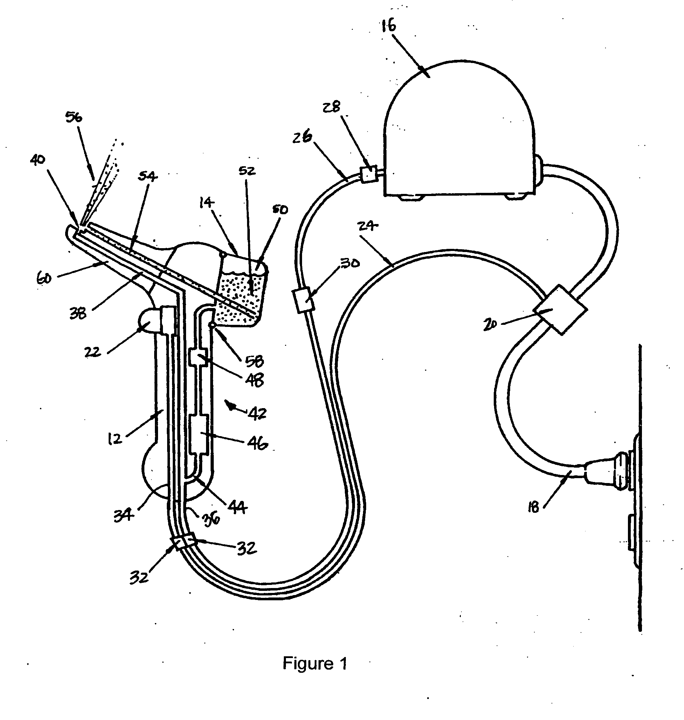

Figure 1 is a diagrammatic illustration of a home dental cleaning appliance according to the principles of the present invention.

### DETAILED DESCRIPTION OF THE PREFERRED EMBODIMENT

The following description of the preferred embodiment is merely exemplary in nature and is in no way intended to limit the invention, its application, or uses.

The dental prophylaxis and air appliance of the present invention uses a cleaning technique that directs an abrasive-laden fluid stream at the teeth, gingiva, and other oral tissue. The abrasive stream cleans out food particles while removing plaque from the surfaces of the teeth and at the tooth/gingiva interface. Additionally, the devices stimulate circulation in the gingiva and oxygenates various anaerobic bacteria, both of which help to prevent periodontal diseases.

With reference to Figure 1, a dental prophylaxis and air appliance (hereinafter "air appliance"), generally indicated at reference numeral 10, is provided according to the principles of the present invention. Air appliance 10 generally includes a hand piece 12, a slurry container 14 coupled to hand piece 12, and a compressed air source 16 in fluid communication with hand piece 12, which will be more fully described below.

Air appliance 10 further includes a power supply cord and adapter 18, which is configured to be operably received in any one of a plurality of standard power outlets available around the world. Power supply cord 18 is electrically coupled to a switch member 20. Switch member 20 is preferably a pneumatically operated switch having a button member 22 extending from hand piece 12 and in communication with switch member 20 via a switch line 24, such that upon actuation of button member 22, switch member 20 permits electrical power to flow to compressed air source 16. However, it should be understood that switch member 20 might be of any conventional design that permits safe and convenience operation of air appliance 10.

Compressed air source 16 is preferably an electrical air compressor capable of providing compressed air via a compressed air line 26. Preferably, an air pressure regulator 28 and a filter/drier 30 are disposed within compressed air line 26 to ensure proper and adequate filtering and pressure regulation of the compressed air.

Switch line 24 and compressed air line 26 are coupled to hand piece 12 via a pair of quick disconnect coupling members 32. More specifically, switch line 24 is coupled to an internal switch line 34 of hand piece 12. Similarly, compressed air line 26 is coupled to an air supply line 36 of hand piece 12. It should be understood that button member 22, internal switch line 34, switch line 24, and switch member 20 constitute a switching system capable of selectively actuating air appliance 10. In addition to compressed air line 26, it should be appreciated that additional lumen lines may be coupled within air appliance 10 to facilitate use of air appliance 10 in various applications. For example, additional single or multi-lumen lines may include a passive drain tube, vacuum tube, air delivery system, water delivery system, medicament delivery system, fiber optic delivery system, or combinations thereof.

Air supply line 36 may be diverted into two paths―an air delivery tube 38, which terminates at a small bore orifice 40 in the tip of hand piece 12; and a slurry pressurization system 42. Slurry pressurization system 42 includes a supply line 44 in fluid communication with an optional filtration system 46 and an air-metering device 48 that terminates into slurry container 14. Air metering device 48 serves to provide a generally constant predetermined air pressure within slurry container 14. By way of non-limiting example, air-metering device 48 may be a needle valve, a sintered or crushed stainless steel tubing, a ruby-orifice, or a microbore port. Pressurized air flowing into slurry container 14 forms a pressure head 50 acting upon a slurry mixture 52 contained in slurry container 14. In response to this pressure head 50, slurry mixture 52 is forced out of a slurry delivery tube 54 at a predetermine delivery rate to form an atomized slurry and air spray 56. This delivery rate may be adjusted depending upon slurry consistency, slurry type, desired flow rate, and the like. Filtration system 46 and filter/drier 30 may be employed in order to provide improved longevity and reliability to air appliance 10. However, these features may be eliminated to reduce cost of production. It should be appreciated that although slurry mixture 52 is presently described as being a liquid mixture, the present invention should not be regarded as being limited to such. A more complete description of alternative agents, materials, and the like that may be used in place of slurry mixture 52 is provide below.

It should be understood that slurry container 14 is removably mounted to hand piece 12 using any one of a number of conventional mounting device, such as, but not limited to, a snap-on fit, interference fit, locking engagement, slidable engagement, screwing connection, and the like. Preferably, a seal 58 is provided between slurry container 14 and hand piece 12 to prevent leakage. However, it is also important to understand that slurry container 14 may be separate from hand piece 12, such that it may reside out of view, with a base unit, or in any other location convenient to the particular application. Additionally, it should be understood that slurry container 14 may be disposable to facilitate its use with a wide variety of materials.

Additionally, it is preferably that hand piece 12 includes a removable spray tip member 60 coupled thereto. To this end, removable spray tip member 60 may have any one of a plurality of shapes, delivery streams, and applications. By way of non-limiting example, removable spray tip member 60 may include a brush, a sponge or rubber scraper, a tongue scraper, a periodontal aid, an upper and lower tray with multiple jets to deliver medium across all teeth simultaneously, or a pneumatic driving power brush system with air/slurry spray. Removable spray tip member 60 is preferably a quick change, 360° rotating individual user tip, which takes on different shapes depending upon the embodiment. It should be understood that hand piece 12 and spray tip member 60 may be one self-contained disposable unit or an assembled unit.

The force produced by atomized slurry and air spray 56 is sufficient to remove food particles, plaque, tarter, and other dental calculus from the surfaces of the teeth, the areas between the teeth, and the tooth/gingiva interface. Additionally, the air and slurry stimulates circulation in the gingival tissues and oxygenates various anaerobic bacteria thereby inhibiting periodontal diseases. Using baking soda as the abrasive slurry also has the further effect of neutralizing the specific environment in which both the aerobic and anaerobic bacteria thrive.

As described above, slurry mixture 52 should not be interpreted as being limited to liquid slurries. As such, any one of a number of material components may be used and are anticipated as being within the scope of the present invention. By way of non-limiting example, air appliance 10 of the present invention may be used in connection with dry materials, premixed slurries, compressed tablets, or even premixed packets or dosages. Additionally, these materials may be used in any one of a number of forms, such as dry, gel, cream, fluid, or colloid suspension. Still further, the actual material used for cleansing and/or treatment may be chosen from at least the following: an abrasive (calcium triphosphate, psylumm husk, etc.), a non-abrasive, an anionic surfactant, a cationic surfactant, a nonionic surfactant, a medicament, a flavoring (mint, fruit, etc.), an astringent (tea tree oil, thymol, etc.), a disinfectant (chloride or chlorite ion, silver compound, sodium chloride, sodium bromide, sodium iodide, potassium chloride, potassium bromide, potassium iodide, magnesium chloride, magnesium bromide, magnesium iodide, calcium chloride, calcium bromide, calcium iodide, etc.), an oxygenating agent (hydrogen peroxide), an enzyme or a coenzyme (papaya extract, coenzyme Q10), a vitamin (alpha-tocopherol (vitamin E), pyridoxal (vitamin B₆), ascorbic acid (vitamin C), vitamin B₁₂, vitamin A, vitamin D, vitamin K, etc.), a mineral (zinc, calcium chloride, etc.), an organic, an inorganic, a sweetener (xylitol, saccharin, carrigin, etc.), a combatant, an antimicrobial (chlorhexidine gluconate, triclosan, etc.), an antibiotic (tetracycline, metrodizole, etc.), a bacteria, a virus, an antiviral agent (acyclovir, etc.), a desensitizing agent (potassium nitrate, acidic oxalate composition, fluoride, etc.), an odor-eating agent (zinc, sodium bicarbonate, etc.), an acid, a base, a neutral, a bleaching agent (carbamide peroxide), an antioxidant, an anti-inflammatory agent (salicylic acid, 4-amino salicylic acid, esters of salicylic acid, esters of 4-aminosalicylic acid, sulfanilamide, etc.), a sealant (waxes, poly-n-vinyl pyrrolidone, crystalline fatty alcohols, paraffins, polyethylene oxides, hydroxypropyl cellulose, and cellulose derivatives, etc.), a coating (glycerin, sorbitol, polyethylene glycol, polyglycerols, propylene glycol, etc.), an anti-tarter agent, an anti-adherent agent, an anti-agglomerate, a remineralization agent (fluoride, calcium, phosphate, etc.), a resorbable polymer (poly(D,L-lactide), poly(D,L-lactide-co-trimethylene carbonate), poly(ethylene-co-propylene), poly(ethylene-co-vinyl acetate), poly(D,L-lactide-co-glycolide), carboxymethylelhyl cellulose, synthetic hydroxyl functional polymers, polyacrylamide, polyoxymethylene, etc.), a copolymer, an astringent (grape seed extract, etc.), a disinfectant (chloride, chlorite ion, etc.), a time-releasing agent (hydroxyethylcellulose, selenium, etc.), a cox1 inhibitor, a cox2 inhibitor, a lipid, a protein, a carbohydrate, an oil alcohol, a phenol agent, a phosphorylated nucleotide (adenosine triphosphate (ADP), adenosine diphosphate (ADP), adenosine monophosphate (AMP), guanosine triphosphate (GTP), uridine triphosphate (UJTP), etc.), an amino acid (aspartic acid, glutamic acid, glutamine, arginine, glycine, etc.), an antihistamine (benadryl), a steroid (predonsone, prednisolone, prednisone, dexycorticosterone, cortisone, hydrocortisone, estrogen, etc.), a disintegrin (monoclonal antibodies, etc.), a glycosaminoglycan (hyaluronic acid, chondroitin sulfate, heparin, heparin sulfate, keratin sulfate, etc.), a growth factor (fibroblast growth factor (FGF), platelet-derived growth factor (PDGF), insulin-like growth factor (IGF), transforming growth factor (TGR), epidermal growth factor (EGF), etc.), a periodontal tissue regenerator (collagen, glycosaminoglycans (e.g., hyaluronic acid, heparin sulfate, chondroitin sulfate) proteoglycans (e.g., versican, biglycan), substrate adhesion molecules (e.g., fibronectin, vitronectin, laminin) etc.), an a hydrophobic layer (silicone oils, modified silicones, etc.).

Additionally, air appliance 10 of the present invention may also be adapted for both professional and home applications. Furthermore, air appliance 10 may be adapted to provide high, medium, low, or no (0) pressures depending upon the needs of the particular application. Still further, air appliance 10 may include an optional air tank ballast (not shown) in conjunction with the current design to provide an increase volume of air over a period of time, a super charger or volumetric reservoir of air. Additionally, other fluid bottles for other components or liquids may be used in conjunction with the present invention.

It should be appreciated from the foregoing that air appliance 10 of the present invention may also be used for dermatological applications, recreational applications, toys, propulsion devices, preservation devices, marinating devices, drain opener, fire extinguisher, lather-maker, pressurized cooking devices, nasal and sinus cleansing applications, humidifying device, nebualizing devices, atomizing devices, manicuring applications, massaging device, air delivering tool devices, and painting devices. However, it should also be appreciated that variation in construction may be necessary to achieve these alternative uses, which may include variations to switch member 20, air pressure regulator 28, compressed air source 16, metering device 48, switch line 24, compressed air line 26, slurry container 14, slurry mixture 52, spray tip member 60, hand piece 12, or small bore orifice 40.

It should be readily apparent that the present invention provide a number of useful advantages over the prior art, such as easily used as a home device due to the lack of a need for a pressurized water vessel. Additionally, the present invention is capable of varying the dilution of the slurry and consequently the level of abrasiveness or efficacy of the cleaning through the amount of water or other liquid used in wetting the dry components.

The description of the invention is merely exemplary in nature and, thus, variations that do not depart from the gist of the invention are intended to be within the scope of the invention. Such variations are not to be regarded as a departure from the spirit and scope of the invention.

## Claims

1. An air appliance comprising:
a compressed air source (16) for supplying compressed air;
a hand piece (12) in fluid communication with said compressed air source, said hand piece having a spray tip member (60), said hand piece operable to receive said compressed air and deliver at least said compressed air to said spray tip member; and
a component material container (14) in fluid communication with said compressed air source and said spray tip member of said hand piece, said abrasive container operable to contain a component material and deliver said component material to said spray tip member such that said component material is at least partially mixed with said compressed air at said spray tip member.

2. The air appliance according to Claim 1, further comprising:
a switch member (20) operably coupled to said compressed air source for selectively actuating said compressed air source to deliver said compressed air.

3. The air appliance according to Claim 2 wherein said switch member (20) is a pneumatic switch.

4. The air appliance according to Claim 1 wherein said compressed air source (16) is an electrical compressor.

5. The air appliance according to Claim 1 wherein said component material container (14) is removably coupled to said hand piece (12).

6. The air appliance according to Claim 1, further comprising:
an air pressure regulator (28) disposed between said compressed air source (16) and said hand piece (12), said air pressure regulator operable to generally maintain a substantially constant predetermined pressure of said compressed air.

7. The air appliance according to Claim 1, further comprising:
a filter device disposed between said compressed air source (16) and said hand piece (12), said filter device operable to filter said compressed air from said compressed air source.

8. The air appliance according to Claim 1, further comprising:
an air metering device (48) disposed between said compressed air source (16) and said component material container (14), said air metering device operable to control a flowrate of said compressed air to said component material container.

9. The air appliance according to Claim 8, further comprising:
a filter device disposed between said compressed air source (16) and said air metering device (48), said filter device operable to filter said compressed air from said compressed air source.

10. The air appliance according to Claim 1 wherein said spray tip member (60) is removably coupled to said hand piece (12) to provide interchangeability with a second spray tip member.

11. The air appliance according to Claim 1 wherein said component material container (14) sealingly engages said hand piece (12), said component material container having an inlet in fluid communication with said compressed air source, said component material container further having an outlet in fluid communication with said spray tip member (60), said component material container being operable to receive said component material (52) so as to define a pressure head forcing said component material out of said outlet.

12. The air appliance according to Claim 1 wherein said component material container (14) is spaced apart and in fluid communication with said hand piece (12).

13. The air appliance according to Claim 1 wherein said component material (52) is a mixture of dry abrasive and non-abrasive components that is capable of defining a slurry when a liquid is added.

14. The air appliance according to Claim 1 wherein said component material (52) is a dry material.

15. The air appliance according to Claim 1 wherein said component material (52) is a premixed slurry.

16. The air appliance according to Claim 1 wherein said component material (52) is a compressed tablet.

17. The air appliance according to Claim 1 wherein said component material (52) is provided in a premixed packet or dosage.

18. The air appliance according to Claim 1 wherein said component material (52) is chosen from the group consisting essentially of gel, cream, fluid, or colloid suspension form.

19. The air appliance according to Claim 1 wherein said component material (52) is chosen from the group consisting essentially of an abrasive, a non-abrasive, an anionic surfactant, a cationic surfactant, a nonionic surfactant, a medicament, a flavoring, an astringent, a disinfectant, an oxygenating agent, an enzyme, a coenzyme, a vitamin, a mineral, an organic, an inorganic, a sweetener, a combatant, an antimicrobial, an antibiotic, a bacteria, a virus, an antiviral agent, a desensitizing agent, an odor-eating agent, an acid, a base, a neutral, a bleaching agent, an antioxidant, an anti-inflammatory agent, a sealant, a coating, an anti-tarter agent, an anti-adherent agent, an anti-agglomerate, a remineralization agent, a resorbable polymer, a copolymer, an astringent, a disinfectant, a time-releasing agent, a cox1 inhibitor, a cox2 inhibitor, a lipid, a protein, a carbohydrate, an oil alcohol, a phenol agent, a phosphorylated nucleotide, an amino acid, an antihistamine, a steroid, a disintegrin, a glycosaminoglycan, a growth factor, a periodontal tissue regenerator, an a hydrophobic layer.

20. A dental prophylaxis comprising:
a compressed air source (16) for supplying compressed air;
a hand piece (12) in fluid communication with said compressed air source, said hand piece having a spray tip member (60), said hand piece operable to receive said compressed air and deliver at least said compressed air to said spray tip member;
a switch member (20) operably coupled to said compressed air source for selectively actuating said compressed air source to deliver said compressed air;
a component material container (14) in fluid communication with said compressed air source and said spray tip member of said hand piece, said abrasive container operable to contain a component material and deliver said component material to said spray tip member such that said component material is at least partially mixed with said compressed air at said spray tip member;
an air pressure regulator (28) disposed between said compressed air source and said hand piece, said air pressure regulator operable to generally maintain a substantially constant predetermined pressure of said compressed air;
a first filter device disposed between said compressed air source and said hand piece, said first filter device operable to filter said compressed air from said compressed air source; and
an air metering device (48) disposed between said compressed air source and said component material container, said air metering device operable to control a flowrate of said compressed air to said component material container.

21. The dental prophylaxis according to Claim 20, further comprising:
a second filter device disposed between said compressed air source (16) and said air metering device (48), said second filter device operable to filter said compressed air from said compressed air source.

22. The dental prophylaxis according to Claim 20 wherein said switch member (20) is a pneumatic switch.

23. The dental prophylaxis according to Claim 20 wherein said component material container (14) is removably coupled to said hand piece.

24. The dental prophylaxis according to Claim 20 wherein said spray tip member (60) is removably coupled to said hand piece (12) to provide interchangeability with a second spray tip member.

25. The air appliance according to Claim 20 wherein said component material container (14) sealingly engages said hand piece (12), said component material container having an inlet in fluid communication with said compressed air source, said component material container further having an outlet in fluid communication with said spray tip member, said component material container being operable to receive said component material (52) so as to define a pressure head forcing said component material out of said outlet.

26. The air appliance according to Claim 20 wherein said component material container (14) is spaced apart and in fluid communication with said hand piece (12).

27. The dental prophylaxis according to Claim 20 wherein said component material (52) is a mixture of dry abrasive and non-abrasive components that is capable of defining a slurry when a liquid is added.

28. The dental prophylaxis according to Claim 20 wherein said component material (52) is a dry material.

29. The dental prophylaxis according to Claim 20 wherein said component material (52) is a premixed slurry.

30. The dental prophylaxis according to Claim 20 wherein said component material (52) is a compressed tablet.

31. The dental prophylaxis according to Claim 20 wherein said component material (52) is provided in a premixed packet or dosage.

32. The dental prophylaxis according to Claim 20 wherein said component material (52) is chosen from the group consisting essentially of gel, cream, fluid, or colloid suspension form.

33. The dental prophylaxis according to Claim 20 wherein said component material (52) is chosen from the group consisting essentially of an abrasive, a non-abrasive, an anionic surfactant, a cationic surfactant, a nonionic surfactant, a medicament, a flavoring, an astringent, a disinfectant, an oxygenating agent, an enzyme, a coenzyme, a vitamin, a mineral, an organic, an inorganic, a sweetener, a combatant, an antimicrobial, an antibiotic, a bacteria, a virus, an antiviral agent, a desensitizing agent, an odor-eating agent, an acid, a base, a neutral, a bleaching agent, an antioxidant, an anti-inflammatory agent, a sealant, a coating, an anti-tarter agent, an anti-adherent agent, an anti-agglomerate, a remineralization agent, a resorbable polymer, a copolymer, an astringent, a disinfectant, a time-releasing agent, a cox1 inhibitor, a cox2 inhibitor, a lipid, a protein, a carbohydrate, an oil alcohol, a phenol agent, a phosphorylated nucleotide, an amino acid, an antihistamine, a steroid, a disintegrin, a glycosaminoglycan, a growth factor, a periodontal tissue regenerator, an a hydrophobic layer.
